# EUROPEAN PATENT SPECIFICATION

(11) **EP 2 470 076 B1**
(45) Date of publication and mention of the grant of the patent: **31.05.2017**
(21) Application number: 10812688.9
(22) Date of filing: 29.08.2010
(51) Int. Cl.: A61B 5/00, A61B 5/103, A61B 5/11, G06K 9/00, A63B 24/00, A63B 71/06, A63B 69/36, A63B 69/38, G06F 19/00, G01S 5/02

(54) **CHARACTERIZING A PHYSICAL CAPABILITY BY MOTION ANALYSIS**
KENNZEICHNUNG EINES PHYSISCHEN VERMÖGENS DURCH BEWEGUNGSANALYSE
CARACTÉRISATION D'UNE APTITUDE PHYSIQUE PAR ANALYSE DU MOUVEMENT

(30) Priority: 28.08.2009 US 238039 P
(43) Date of publication of application: 04.07.2012
(73) Proprietor: Selner, Allen Joseph, Manhattan Beach, California 90266 (US)
(72) Inventor: Selner, Allen Joseph, Manhattan Beach, California 90266 (US)
(74) Representative: Fennell, Gareth Charles
(86) International application number: PCT/US2010/047067
(87) International publication number: WO 2011/026001

(56) References cited:
- WO-A1-2007/029012
- WO-A2-2009/053901
- US-A- 5 111 410
- US-A- 5 891 060
- US-A1- 6 056 671
- US-A1- 2004 220 490
- US-A1- 2005 240 086
- US-A1- 2008 045 804
- US-A1- 2008 306 980

## Description

### TECHNICAL FIELD

Embodiments of this invention include the analysis of human movements to assign a designation of capability in a physical activity domain.

### BACKGROUND ART

Kinematic and kinetic measurements have been made for the purpose of understanding human physiology, for diagnosing disorders, for sports study, and for sport performance improvement. Movement data has been collected by a variety of measurement techniques including by devices attached to the body, and by cameras detecting movement of body parts, and by detecting movement of specially marked points on a body.

Specialized sports, clinical, and research use of this technology have included the coaching of elite athletes, predicting the later appearance of Cerebral Palsy symptoms in infants, and tracking improvements over a course of treatment. Costs of dynamic body motion and force measurement devices have lowered and biomechanical knowledge has increased. However, the wide array and complexity of possible human motions, the large amount of raw data generated, and particularly a lack of results that are useful without expert interpretation, have significantly limited the routine exploitation of the tools and techniques of this field. Inexpensive and routinized solutions to incorporate motion-based measurements into everyday health care can have a great importance in overall cost control. Previous work has included WO 2007/029012 "Categorizing Movement Data"; US 6,248,063 "Computer Assisted Methods for Diagnosing Diseases"; US 5,533,519 "Method and Apparatus for Diagnosing Joints"; "The Gait Profile Score and Movement Analysis Profile", Baker Richard, et al. Gait Posture 2009; "Extracting a Diagnostic Gait Signature" Lakany, Heva. Pattern Recognition 41 (2008); "Applications of Artificial Neural Nets in Clinical Biomechanics, Schollhorn, W.I. Clinical Biomechanics 19 (2004).

US patent application no US2005240086 describes an intelligent wearable monitoring system that includes a wireless personal area network for extended monitoring of a patient's motor functions. The wireless personal area network includes an intelligent accelerometer unit, a personal server and a remote access unit. The intelligent accelerometer unit measures acceleration data of the patient, in real-time. The personal server processes the acceleration data, applying linear and non-linear analysis, such as fractal analysis, to generate motor function information from the acceleration data. Motor function information is transmitted to a remote access unit for statistical analysis and formatting into visual representations. A data management unit receives the formatted motor function information and displays the information, for example, for viewing by the patient's physician.

International patent application no WO2009053901 describes a method for determining a viewing configuration of a rendered figure of a rehab-patient or a sports trainee, aiming to deliver a suitable view on the rendered figure, the method comprising the steps of capturing motion data in the 3D space of one or more body parts of the rehab-patient or the sports trainee and providing them to a computer; and further the step of performing on the computer measurements of deviation of the captured motion data from a reference list of motion data and/or measurements of main motion direction; and based on the results of the above measurements, determining the viewing configuration.

US patent application no US2008045804 describes a method or system involving associating a plurality of biokinetographic comparison results with a first specific dysfunction from a group of specific dysfunctions, each of the biokinetographic comparison results obtained from a comparison of a biokinetographic value to a standard for a corresponding biokinetographic variable.

US Patent No. 5111410 describes a motion diagnosis system which picks up motions of a subject and analyzes them, and is characterized by measuring address data at points of images thus picked up according to a trigger condition set in advance, displaying the result of the measurement in graphic images or stored images or in comparison with reference images, outputting the evaluation of such comparison to thereby enable measurement under flexible trigger conditions and analyzing the motions of the subject based on quantitative data in a manner easily understandable by the viewers and simple operation.

US patent application no US2008306980 describes a system and method used to assess animal behavior that includes a module having sensors that collects a variety of physical and biological data from a test subject. Interpretation of the data is provided to assess the test subjects behavior, neurology, biochemistry and physiology. The module is useful in observing the effects of a drug on the test animal and providing information on the drug's signature. Another advantage is the module's portability that allows it to be used in standard laboratory cages. This portability allows the animal to be tested in its own habitat, that can reduce any erroneous data due to stressing the animal when removed to a test cage. Additionally, the module's design allows for parallel data collection and interpretation from several laboratory animals undergoing different experiments.

### SUMMARY

There are sources of movement data collection that can provide volumes of information from instrumented movements. The teachings herein can make biomechanical data relevant to clinicians and coaches by producing and using protocols that can provide understandable ratings relevant to a physical task of interest. Motion test protocols can advantageously be administrable by modestly trained individuals and provide results rapidly and preferably relatively automatically.

Methods and systems taught herein can include an ordinal or scalar rating or an objectively defined discreet classification. These ratings and classifications can be of a physical capability or physical performance based on measurements made during performance of prescribed movement protocol. A set of predetermined, relevant movement-related information can be collected for analysis. The collected information can be analyzed in light of predetermined criteria to produce an objective classification or rating. Various applications of these teachings can have distinct executable movement and measurement protocols. Data mining techniques can be used over data representation of a large group of individuals to identify key parameters of movements to allow unknown subject's to be classified. Methods and systems for performing tests and producing a quantified rating of subjects, as well as methods and systems for creating such protocols, are within the teaching herein.

### PROBLEM

A problem solved by the teachings herein is the creation of efficient and reliable semi-automatic analysis of large quantities of motion-related data from a human into a form for readily quantifying a particular physical capability in comparison to other humans. The quantifying might be on a numeric scale.

### BRIEF DESCRIPTION OF DRAWINGS

The various drawings are to better illustrate the concepts described herein and to better teach those skilled in the art to make, use, and carry out these teachings. They are not intended to be limiting or to set metes and bounds.
FIG. 1A shows a human in a schematic view and indicates examples of some motions that might be included in developing a protocol;
FIG. 1B shows a human in a schematic view and a further example of a motion that might be included in developing a protocol;
FIG. 2A illustrates one possibility for instrumentation of a human's motion involving a body suit with embedded motion and direction sensors;
FIG. 2B illustrates one possibility for instrumentation of a human's motion using visual markers in various body locations viewed by multiple cameras for 3D position determination;
FIG. 3 is a flow chart of the data collection steps involved with an example of protocol creation;
FIG. 4 is a flow chart of the data mining and analysis steps involved with an example of protocol creation;
FIG. 5 is a flow chart of the data collection steps involved with an example of protocol execution;
FIG. 6 is a flow chart of the data mining and analysis steps involved with an example of protocol creation;
FIG. 7 illustrates a system for performing a protocol for evaluating a tennis swing; shown are a human subject with attached accelerometers at selected locations, a data capturing and preprocessing computer, and a data analysis computer.

### DISCLOSURE OF INVENTION

### Introduction

Methods and Systems for developing and using evaluative test protocols are described by way of example embodiments. By protocol, as used herein, generally mean (a) a preplanned set of steps or actions to gather particular information related to a subject, student or patient; (b) a preplanned series of steps and actions to analyze, scale, compare, or transform that "raw" information and; (c) a predetermined method and criteria for assigning a rating, score, index or labeled classification based upon the information analysis. While a full protocol would include the steps of (a), (b), and (c) above, the term protocol can signify these steps individually. A trivial example of a protocol would be the steps for taking someone's blood pressure. A more complex protocol might be the series of steps involved in preparing a patient, configuring equipment, and administering an MRI scan.

Data mining includes mathematical and computational techniques of unstructured analysis and correlation between multiple parameters. These techniques help to uncover unexpected relationships between the parameters. When the data forms in relatively tight groupings those groupings can be called clusters.

The examples presented include both methods for developing a specific protocol and the performance of those executable protocols to transform motion data representative of an individual's performance into a readily understood classification or figure of merit.

### Examples

The many areas of relevant human physical capability assessment include, for example: sports training, occupational choices, geriatric assessments, medical treatment progression, and malingering detection. Other areas of human physical activity relevant to the teachings herein include physical therapy, exercise routines, and game playing. Despite the availability of low-cost motion and force sensors and a rich understanding of biomechanics, assessments are nonetheless routinely made in a subjective manner or by static measurements such as range-of-motion. Alternatively, there are motion labs that can produce volumes of real-time raw motion information from which it may be difficult to draw conclusions. Some automatic and semi-automatic methods and systems applying these teachings have the potential to significantly lower a wide set of health care costs by adding quantized motion-related measurements to everyday medical care.

Examples of the teachings herein can use data mining techniques to semi-automatically analyze a set of collected movement and non-movement related parameters. That analysis can determine the statistical significance of each member of the set of parameters, in regards to a correlated attribute. A subset of more significant tests from the original comprehensive set of information can be identified for incorporation into an efficient executable protocol on a Pareto principle basis.

### Method Overview

Embodiments of protocols for objective, repeatable, and quantified ratings based on kinematic, kinetic and other data can be developed by:
(1) Applying subject domain knowledge to postulate a comprehensive universe of movements and a comprehensive universe of collectable data to be representative of parameters of those movements.
(2) For a set of subjects with known attributes in the particular domain of interest: directing subjects to perform the predetermined universe of movements while instrumented to collect data regarding the universe of parameters.
(3) Analyzing the collected data with linear and non-linear mathematical and computational methods. Those methods can include: multivariate regression, neural networks, and data mining by classification, clustering, self-organizing maps, and other approaches. The goal of the analysis can be to find parameters that correlate with the subjects' pre-known capabilities.
(4) Organizing the parameters by their predictive power or correlative strength.
(5) Building up a list of movements and their respective parameters to produce an executable protocol from a sub-set of the original universe of movements. The subset collectively having a desired level of overall predictive and correlative power.

Protocols consistent with the principles herein can also involve measuring non-motion parameters such as EMG (Electromyogram) and Ground Reactive Force (GRF) information and can also involve static variables such as body type, demographic, physiological, static biomechanical factors, and psychological information.

### Example 1 - Back pain assessment

In the case of back pain assessment, a set of subjects with known, varying degrees of impairment are tested.

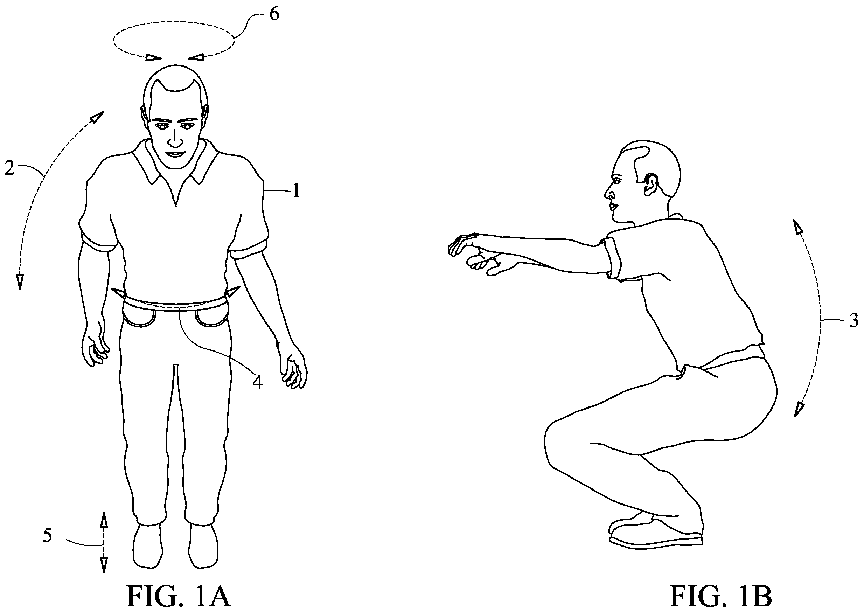

With some clinical insight, a comprehensive set of motions are prescribed for subjects to perform while a comprehensive set of parameters characterizing those movements is collected. Figures 1A and 1B illustrate a person 1 and a variety of possible prescribed motions. For example, subjects may be instructed to bend in one or more specific directions 2, to stand from a sitting position 3, to twist body portions 4 at various rates, to walk normally, and turn or move various body parts 5 6. Motions might be repeated multiple times. Prescribed motions might comprise motions that are performed under load and those that are not loaded or with a different degree of load. Both motions that involve biomechanically open kinetic chains and those that involve closed kinetic chains might be used.

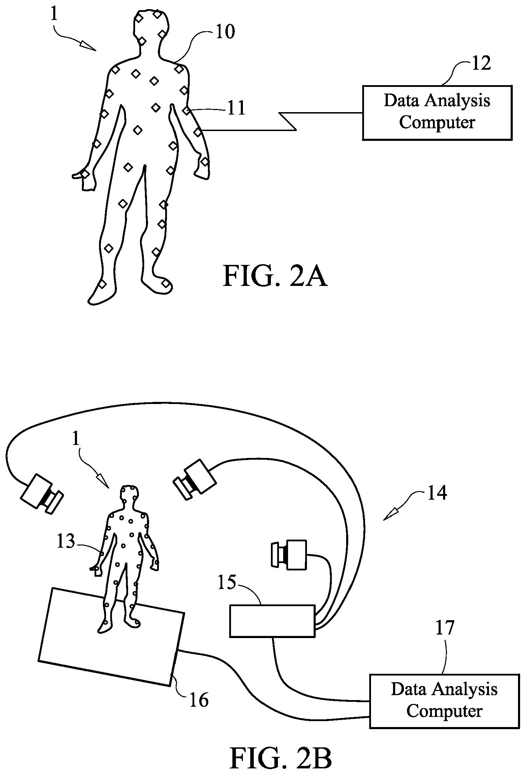

Those skilled in the art will recognize that there are many ways to collect human movement related parameters. Figures 2A and 2B illustrate alternate methods of 3D real-time full-body instrumentation. Figure 2A schematically represents a person wearing a full body suit 10 and points out locations of embedded motion and direction sensors 11 that communicate with a data analysis computer 12. Figure 2B, in contrast, shows a person with a plurality of marked spots 13. A multi-camera system 14 provides an apparatus to track the location of each spot in real time via a 3D tracking system 15. Some versions use passive spots and others can use actively light emitting spots. The tracked spot data and data from a pressure plate 16 are analyzed by a data analysis computer 17. In addition to positions in space, other parameters such as changes in joint angles and EMG data could be determined as well.

Each of the subjects with known capabilities or known impairments performs the determined set of motions while instrumented. The information is accepted and stored. The large volume of information resulting from the above tests is analyzed with non-linear techniques including artificial neural nets (ANN), self-organizing maps (SOM), machine learning classification trees, fuzzy classification, and other data mining techniques. Analysis by regression, multivariate analysis, and other more traditional statistical methods may be employed.

These analyses can produce a clustering of the various subjects' performance into discreet classifications or can find correlative statistical significance between the parameters and the known categorization of capabilities of the various subjects. An additional step is to then produce a subset of the initial motions and initial parameters that are particularly sensitive and have statistically significant power in indicating a classification membership or a rating. As those familiar with the art will understand, this is accomplished by further statistical analysis to identify the motions and the parameters associated with those motions that have the greatest predictive power in associating an individual with a cluster, a classification, or a rating. Starting from the most sensitive motion and related parameters on down, a list of motions and measurements is compiled for potential addition to an executable protocol until a desired balance between ease of protocol administration and statistical reliability is achieved. That list of motions and their associated salient parameters become the basis for an executable protocol. Rather than strictly using the top ranked motions and parameters as the basis of an executable protocol, tradeoffs between predictive power and ease of performing the various movements and measuring the various parameters may be made as well. A protocol can be devised that makes trade-offs between time to administer, cost, and complexity of instrumentation, versus confidence in a test's conclusions.

In the case of back pain the subjects have a range of back pain of known severity including some with no back pain. That information is compared to the collected movement parameters. The resulting protocol is intended to produce an overall measure of back impairment or back health that might be used to objectively assess progress over a course of therapy. For back pain assessment or for general back performance capability, a scalar index of 1-10 can apply.

### Example 2 - Malingering assessment

A second application example, also related to back pain, is a protocol for detection of malingering or "sincerity of effort". Rather than result in a scalar index of back health this protocol produces a two-state classification of insincere/sincere effort or faking/not-faking within stated confidence levels. Following the teaching herein, a range of possible movements and measures of those movements were postulated and information regarding those particular factors was measured and analyzed for both actual back pain sufferers and for control subjects. One hypothesis of this assessment was that chronic back pain would result in a fairly consistent motion characteristic as the "point of pain" was entered in performing a prescribed movement. In other words, if a subject was asked to perform a task that resulted in back pain, he or she would experience it at the same point in the movement each time a task was performed. Furthermore it was also hypothesized that the subject might begin to slow down at the point of pain and then be able to accelerate again after the pain diminishes. Subjects were instrumented using a Lumbar Motion Monitor (an exoskeleton attached to the back that can measure range of motion, velocity and acceleration in all three planes of motion). Several movement tasks requiring forward flexing and then extension and finally returning to the starting position were devised. This cycle was divided into 360-degrees so that each trial could be time-normalized to other trials on a position by-position basis. Using the LMM, it was established that movement information could be captured and stored including peak acceleration, average acceleration, peak velocity, average velocity as well as consistency. Other variables such as height, weight, length of limbs, position of foot, anthropometrical details, and other biomechanical factors of each individual were added to the collected data.

Two groups of subjects were tested. One was a group of 19 patients with chronic back pain. This fact was established both by history and by a physical performed by a physician. The second group of 20 had no history of back pain. Both groups were asked to perform the predetermined movement protocol tasks as best they could with full effort. Each group was then asked to repeat the same movement tasks, this time "pretending" they had back pain at a specific location in an attempt to convince us that they had real pain at that location. The goal was to find a group of variables that are readily measureable and, taken together, can reliably place an unknown individual into the correct group.

Over 100 movement and static variables for each subject were derived from the measurements. A statistical regression analysis, consistent with the teachings herein, was performed to see if any subset of these parameters, in combination, had enough predictive power to result in clustering of data that reliably placed a subject into the correct group (faking or non-faking). The analysis produced a formula with a 91 percent chance of placing an unknown individual into the proper group. The most salient factors were related to abruptness of change in acceleration near the point of pain and the consistency of that measure. The "fakers" did not produce the acceleration/deceleration profile at the point of "pain" to a degree and with consistency as to location and as to timing when compared to those with actual back disorders.

An executable protocol was developed to particularly instrument, compute, and evaluate the acceleration/deceleration profile at the point of inflection. This protocol could quickly and reliably categorize sincere and insincere self-reporting of back pain.

### Example 3 - Golf performance index

A sport performance example consistent with the principles taught herein is a "Golf Performance Index". GPI score is a scalar rating of overall level of performance in a golf skill. One way to think about this is as a process for transforming data comprising a time-series of values representing human motions into an objective meaningful measure providing that person's golf swing rating. While learning a new swing a subject may be progressing steadily in their mastery of that new skill but in fact be producing erratic end-results. To coach or to self-coach, an objective measure of progress in learning that swing other than by ultimate outcomes can be valuable. Determining an overall figure of merit of a swing execution based on minimal measurements (for cost reasons and to reduce the intrusive instrumentation borne by the golfer) is desired. A figure of merit or rating achievement of a desired swing can give more valuable feedback to a student than the final outcome of ball flight or golf score. These final outcomes are unduly affected by very small differences in execution or in external factors. Initially a comprehensive set of parameters that may contribute to the accuracy of a golf swing's result is postulated. Motion information is collected over a set of golfers representing a wide range of abilities. Also ball flight accuracy is measured. Those skilled in the art will understand that this can be accomplished either by golfers actually hitting balls or by a virtual golf simulation.

Correlations are determined between each motion parameter, combinations of motion parameters, and ball flight. This can be accomplished by classical linear regression techniques or by data mining techniques including clustering. As in other examples, this mathematical analysis can rank the various motions and measurements of the comprehensive set by their respective statistical predictive power. Keeping practicality of measurement in mind, a subset of motion parameters with effective predictive power is listed and forms the framework of an executable protocol. The number of parameters from the initial set that end up in the executable protocol is based on a desired degree of statistical confidence.

### Example 4 - Occupational Assessment

Rather than describe this example in detail, below is presented a problem amenable to attack by the methods taught herein.

An objective measurement of the capability of making particular job-related movements, particularly under load, is valuable in assessing workers. Periodically, employees can be tested as part of a program to promote safety and health as well as to assess the job-readiness of employees recovering from injury. In cases of recovering from disability, for example, an employee may be deemed ready to return to work when they have regained their pre-injury level in the relevant physical capability. Being able to objectively measure the employees in a job category and then know what level a particular person should be restored to before returning to work would save money and time.

### Example Method of Creating Protocols

A method for developing an executable protocol includes the steps:
1) Accepting a specific domain of interest.
2) Selecting a comprehensive set of real-time motion-related parameters relevant to the domain of interest and selecting other variables to be measured or surveyed.
3) Accepting and storing data from multiple runs of performance with various subjects while those subjects perform the comprehensive set of movements while the comprehensive set of parameters are collected.
4) Analyzing resulting data by at least one of the following techniques: (a) non-linear data mining techniques to find classification clusters, decision tree classifiers, or ordinal, or scalar, or vector rating; and (b) classical statistical methods to determine correlations and other statistically valid relationships to allow meaningful classification or scalar or ordinal indices to be discovered.
5) Ranking the movements and the parameters of those movements by their statistical predictive power.
6) Selecting a subset of the comprehensive movements based on their ranking to comprise the movements and parameters of an executable protocol having a desired level of statistical reliability in classification or rating.

### Protocol Method

Example method for administering a protocol by the steps of:
1) Instrumenting subject for predetermined, real-time motion-related measurements.
2) Performing, by a subject, a predetermined motion sequence. Collecting data regarding a predetermined set of parameters.
3) At least a subset of the collected motion information is formatted for computerized statistical analysis to produce a result that is (1) a classification or (2) an ordinal or (3) scalar rating.
4) Optionally permit real-time viewing of protocol performance by a remotely located monitor.
5) Optionally store video documenting protocol performance along with the data for later verification of correct protocol administration.

The data analyzing step can be performed at a different location and time than the actual testing of the subject.

### Comprehensive Parameters

Those with subject matter knowledge in the application domain of interest may advantageously postulate an initial comprehensive set of factors. Preferably motions, measurements, and derived parameters to be "in the mix" of initial widely constituted measures can include: higher order quantities such as velocity and acceleration, consistency of performance in repeated motions, and angle/angle comparisons of pairs of coordinating body structures. Both motions constituting open kinetic chains and those constituting closed kinetic chains and those unloaded and loaded are also preferably in the initial comprehensive set of parameters. Spectrally pre-processed data, in addition to time-domain data may bring correlative relationships to light. In some cases it is preferred to also include body type, demographic, and psychological variables.

### Programmed Computer Systems

For analyzing of measured and calculated parameters, data mining workbench software such as Weka, Orange, Matlab, IBM DB2 Intelligent Miner and statistical software tools such as SPSS can be used.

### During Protocol Creation

Example method steps performed on data recording computer systems:
1) Filtering and normalize data.
2) Computing predetermined parameters from raw data (velocity determined from acceleration or kinetic information from kinematic information, for example).
3) Packaging data for use by an analysis system.

### Data Analysis Computational Techniques - Protocol Development

One goal of some aspects of protocol creation herein is to discern a subset of motions and subset of possible data measures of those motions to be automatically or semi-automatically analyzed during protocol execution. These subsets would be selected to include data of effective power to provide a protocol that achieves a desired trade-off in ease of administration and the statistical validity of result.

Traditional statistical techniques useful in the data analysis steps include regression, multivariate analysis, and principle component analysis (PCA). Those skilled in the art will be familiar with these mathematical approaches. They are shown applied in this art in US Patent 6,056,671, Marmer; and Quantitative assessment of the control capability of the trunk muscles during oscillatory bending motion under a new experimental protocol, Kim, Parnianpour and Marras, Clinical Biomechanics vol. 11, no. 7, 385-391, 1996. Both references are hereby incorporated herein by reference in their entireties.

In many cases, the powerful, non-linear techniques of data mining including training artificial neural nets (ANN), self-organizing maps (SOM), machine learning classifier trees, and fuzzy decision trees are comprised in the data analysis. Those skilled in the art will be familiar with these computational approaches. They are shown applied in this art in US Published Patent Application 2005/0234309, Klapper, in US Patent 5413116, Radke et. al., and in US patent 6,248,063, Barnhill. All three of these references are hereby incorporated herein by reference in their entireties.

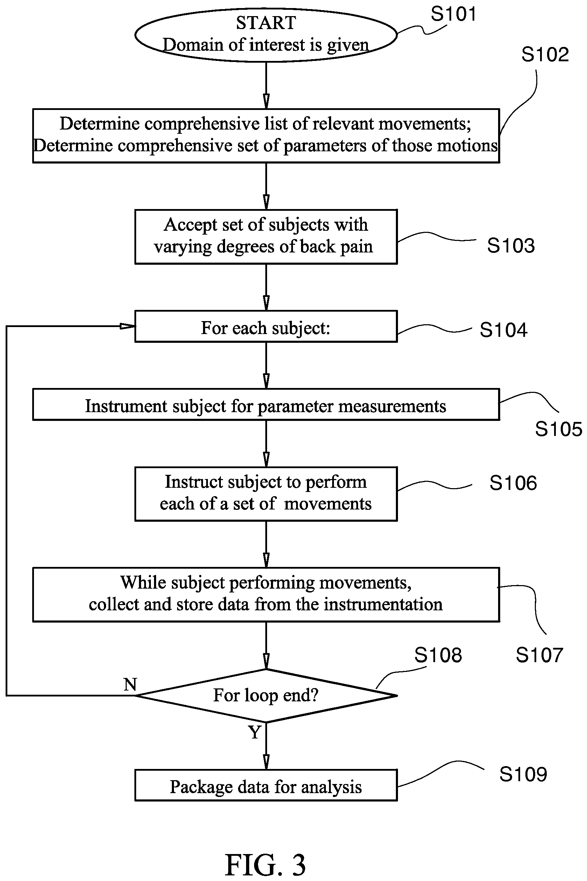

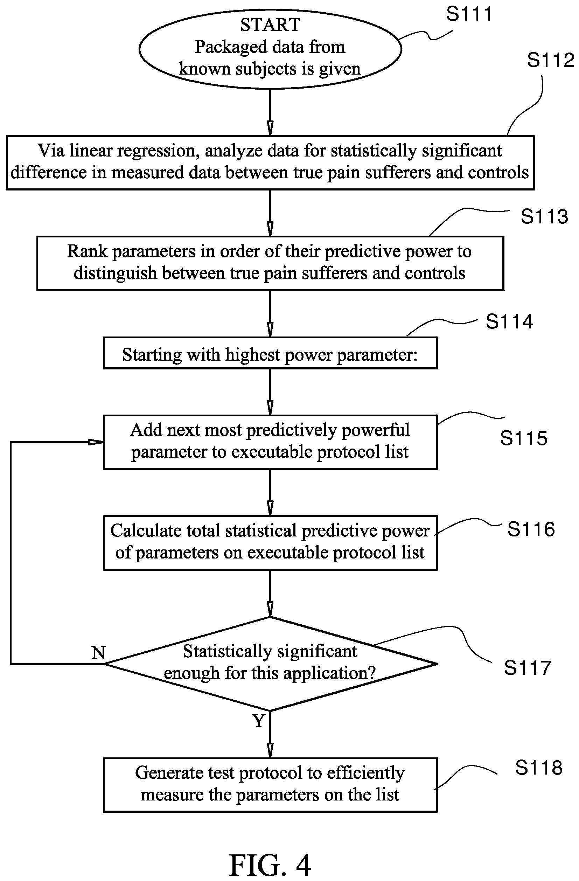

In many implementations consistent with these teachings the initial data is from a large number of subjects with known attributes relative to the physical domain of interest. In other cases, for example with the use of "unsupervised" ANN or clustering techniques, there may not be subjects of known, quantified capabilities. For some protocol creations it may be advisable to have subjects of a known condition as well as "normals". In other cases one might have a subject population that is selected from subjects all suffering from a common condition but in varying degrees. In protocols for tracking changes in an individual, they are analyzed in light of their own performance at various stages of recovery, deterioration, learning, or circumstances. Figures 3 and 4 show flow charts of steps for creating an executable protocol.

Figures 3 and 4 together illustrate one example process for producing a protocol for distinguishing between individuals with back pain and individuals feigning back pain. The first step is determining or receiving a physical motion domain of interest S101. In this case back pain self-reporting veracity is the subject. In the next step a wide range of motions and measurements of those motions is postulated S102 as relevant to making the desired distinction. In the case of back pain, the rate and extent of spine movement is thought to be highly relevant. A Lumbar Motion Monitor that can measure position, velocity, and acceleration of the spine is selected to provide the raw data. The Lumbar Motion Monitor measures in the sagittal, lateral, and twisting planes. A set of subjects with an appropriately wide range of back problems is recruited and selected S103.

In creating the protocol, each subject selected goes through the same set of steps. In FIG. 3 this is expressed by initiating a FOR loop S104. The next steps involve instrumenting the subject at hand S105 according to the previously determined instrumentation and directing the subject to perform the previously determined motions S106. While the subject is performing, those movements', raw data associated with those movements is collected from the instrumentation and stored in a computer readable media S107. The end of the FOR loop for that subject is reached and if there are untested subjects S108 it is decided to return control to the top of that loop S104.

When it is determined that the last subject has been tested S108 the collected and stored data is packaged for analysis S109. This packaging might involve adding non-motion information, providing a copy of the data, or an address within a computer readable media to locate it. More frequently it will involve preprocessing the information to filter out noise and non-meaningful data. It also might involve norming, using principal component analysis for simplification of further data manipulation.

The sequence continues, as shown in FIG. 4 by taking the packaged data as a starting point S111. In this case the next step is to analyze the data using linear regression for variables in the packaged data having high correlation to the pre-known state of the individuals S112. In other, protocols consistent with these teachings, many other forms of analysis can be used. Data mining techniques like cluster analysis as well as artificial intelligence techniques including artificial neural networks might be used in this step.

In the case of a traditional statistical method such as linear regression, the various measured parameters will each have a correlation coefficient or other statistical confidence measure. The next step ranks the various parameters by that statistical quantity S113 with the most predictive first. In a version using cluster analysis, the parameter list might include a list of various subsets of the total, each subset in order by its power. Starting with the first parameter S114 the program module loops through the parameters from top down adding them S115 to the list being compiled of candidate measurement for the protocol being created. As each new parameter is added to the candidate list, the predictive power of the items on the list, taken together, is tested against the received packaged data S116. In FIG. 4 it is seen that this loop is ended when the candidate parameter list reaches a level that is deemed of a high enough significance S117 for the purposes of the protocol being created. In fact many processes consistent with the teachings herein will also assign a rating representing the practicality of making each type of measurement. This can allow optimization for foolproof-ness to administer or time to administer, for example. Therefore the cutoff as to significant-enough S117 could be set conservatively in order to allow for some "candidate" parameters to be rejected for use in the finally produced protocol due to protocol considerations.

After the list of parameters is established, a detailed plan to make those measurements efficiently using cost-effective instrumentation is created S118 which constitutes the basis of a protocol for; in this case, assessing the presence of back pain regardless of a subject's self-reporting.

### During Protocol Execution

### Example Data Collection Steps

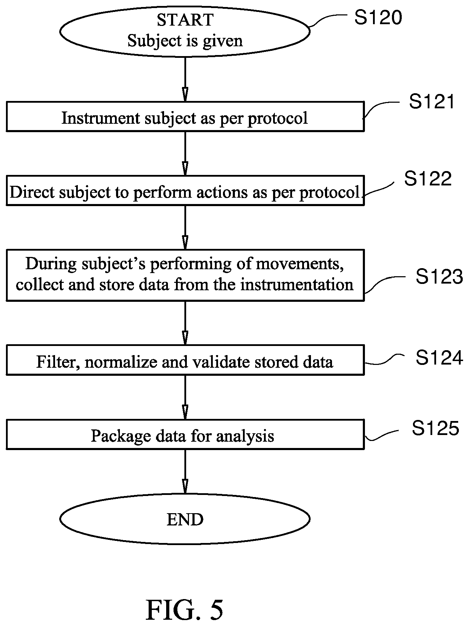

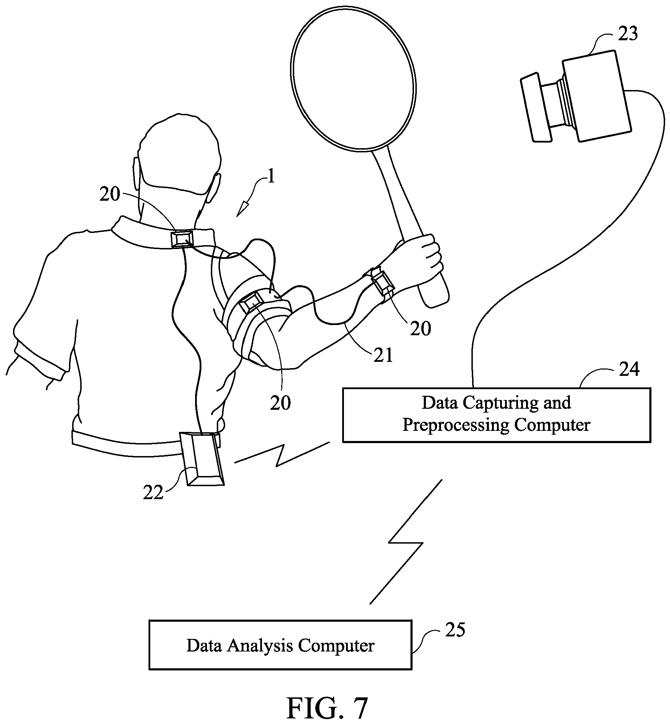

Figure 5 shows a flowchart of the data collection steps of an example executable protocol. Instrumentation that might be used in a tennis application is illustrated in FIG. 7 and further discussed herein.

### Example Data Analysis Steps Performed by a Data Analysis Computer System

1) Accepting movement and force information from pre-processing system.
2) Doing at least one of: (a) applying predetermined decision tree (b) feeding data to trained machine learning data structure to determine classification or rating (c) applying linear or non-linear model.
3) Based on step 2 determine closet match under predetermined rules and set as a rating.
4) Outputting rating.

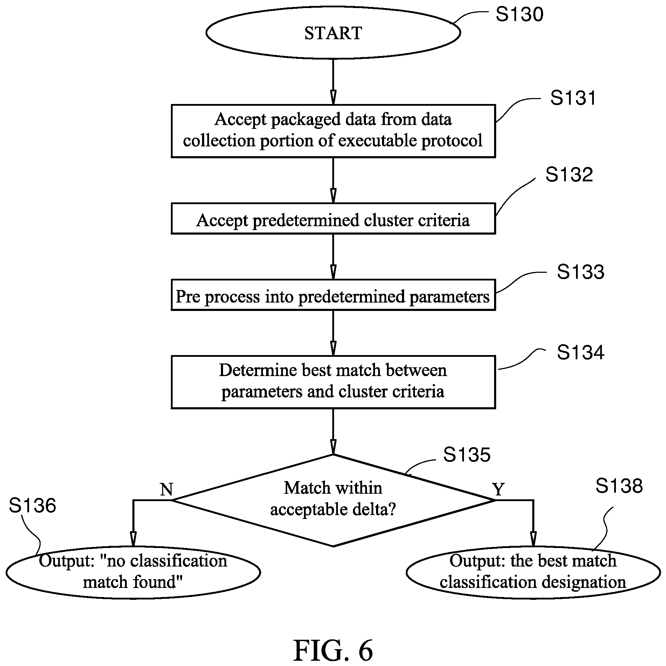

A flow chart illustrating an example of the steps involved in data analysis during protocol execution is seen in FIG. 6.

### Data Analysis Computational Techniques -for protocol execution

The data produced while testing an unknown subject may be first pre-processed to extract pre-determined features. The data may be normalized in one or more dimensions. A rating or categorization may be assigned by linear calculation, by following a classification tree, or by providing data to a trained learning machine.

Figures 5 and 6 represent flowcharts of executing a predefined protocol for assigning an "unknown" subject to one of multiple pre-identified clusters. Figure 5 covers activities directly related to testing the individual while FIG. 6 relates to steps taken to analyze the information from the tests.

The first step of FIG. 5 is receiving S120 the given subject. The next step is to instrument that subject S121 for motion measurements as dictated by the specific protocol being performed (back pain, tennis swing, gait, etc). A prescribed series of motions is performed S122 by the subject while the motion data is being electronically recorded S123 on suitable machine-readable media. Preprocessing steps that might be performed at the time and place of the test include filtering, normalizing, and extracting biomechanical information S124 from the recorded raw data transforming it into a reduced and more meaningful state. Presuming that the analysis is done separately from the information collection, the information is then suitably packaged S125 for analysis. As shown, the analysis is optionally performed at a different location on a different computer system.

The information transformation and analysis of that data is shown in FIG. 6. After the data is accepted S131, the clustering criteria to be applied to that data is accepted S132. In this example, the accepted, preprocessed data is further processed S133 to extract salient features specified by the clustering criteria to use as inputs for cluster match determination S134. That cluster-matching step involves a computer-implemented, mathematical comparison of the salient features of the accepted data to pre-defined, denoted, clusters of parameters specified in the accepted cluster criteria.

If the transformed information representing the subject's motions is found to conform to a denoted cluster with a predetermined degree of statistical acceptability S135, the denotation of that match is output S138. In the case that the measured motion data of the subject does not align with any predetermined cluster to an effective degree, the output is that no classification is clearly indicated S136 by the data.

### System

An example system for executing a protocol consistent with the principles taught herein is shown in FIG. 7. A person 1 is instrumented in a minimal fashion with accelerometers 20 in three chosen locations. The accelerometers are coupled via cables 21 to a belt-mounted controller 22. The controller communicates by Bluetooth compliant wireless signals with a local data capturing and pre-processing computer 24 for recording, initial filtering, normalization and formatting. In one respect, the data capturing and pre-processing computer follows instruction to act as an intelligent electronic recorder. In turn that system communicates with a remotely located analysis and rating system 25. An alternate example system can comprise, as its hardware portion, a home console gaming system such as a Wii with motion and force sensor inputs. Portions of a system can comprise one or more devices like an iTouch or iPhone, which can have accelerometers, GPS, and computational capabilities.

### System Variations

Other system versions might instrument the human with an upper body, lower body or full body suit such as suits in the MVN brand product line offered by Xsens Technologies B.V. and illustrated in FIG. 2A. For some applications the motion or force sensors of a home game system's input device or those in a portable device such as an iTouch may be adequate to make the measurements.

The data preparation system and data analysis and rating system might be remote, might be co-located, or might be implemented on a single computer server. The computational devices used to carry out the method could be a personal computer. In some versions, the system might prompt the subject to perform the limited set of motions. This might be via a text display, by spoken output, or preferably by a video demonstration. In addition, a particular system version could provide a warning that a sequence of motions was not performed as per-protocol and inform the subject or clinician. In some cases, the subject and the computer performing the analysis might not be co-located. At a central facility for data analysis computation, trained computer learning systems, and expertise may serve many protocol execution sites. A camera 23 might be used to capture still or video images of the protocol execution to be stored along with the motion data for future verification of correct protocol administration.

Some implementations based on these teachings may be broken down into foolproof steps, figuratively a "paint- by-numbers" execution protocol. At another time and location more trained personnel can carry out other steps of data analyzing and assignment of discrete classification or of a rating. Below is a pseudo code "flowchart" of an example protocol execution for rating a tennis swing.

### Pseudo Code of Protocol Execution

### START

Instrument Subject, in a predetermined manner for position, motion and force sensing; Operatively couple sensing equipment to data capture computer;
Initiate information capturing by sensors and data capture computer;
Direct subject to perform predetermined tennis swing motion sequences while capturing motion and force and position information;
Capture photographic information of subject while subject is performing predetermined motion sequences;
Command data analysis computer to pre-process captured information and format and package for analysis;
Operatively communicate formatted and packaged information from data capture computer to data analysis computer;
Command data analysis computer to statistically compare information communicated from data capture computer to a database of a set of predetermined quantitative criteria of movement performance;
Is there statistically significant agreement of packaged information and predetermined criteria?
   TRUE: Output numerical rating associated with criteria match
   FALSE: Output: "No reliable match found"
   END

The various illustrative program modules and steps described in connection with the embodiments disclosed herein may be implemented as electronic hardware, computer software, or combinations of both. The various illustrative program modules and steps have been described generally in terms of their functionality. Whether the functionality is implemented as hardware or software depends in part upon the hardware constraints imposed on the system. Hardware and software may be interchangeable depending on such constraints. As examples, the various illustrative program modules and steps described in connection with the embodiments disclosed herein may be implemented or performed with an application specific integrated circuit (ASIC), a field programmable gate array (FPGA) or other programmable logic device, discrete gate or transistor logic, discrete hardware components, a conventional programmable software module and a processor, or any combination thereof designed to perform the functions described herein. The processor may be a microprocessor, CPU, controller, microcontroller, programmable logic device, array of logic elements, or state machine. The software module may reside in RAM memory, flash memory, ROM memory, EPROM memory, EEPROM memory, hard disk, a removable disk, a CD, DVD or any other form of storage medium known in the art. An example processor may be coupled to the storage medium so as to read information from, and write information to, the storage medium. In the alternative, the storage medium may be integral to the processor.

In further embodiments, those skilled in the art will appreciate that the foregoing methods can be implemented by the execution of a program embodied on a computer readable medium either tangible or intangible. The medium may comprise, for example, RAM accessible by, or residing within the device. Whether contained in RAM, a diskette, or other secondary storage media, the program modules may be stored on a variety of machine-readable data storage media such as a conventional "hard drive", magnetic tape, electronic read-only memory (e.g., ROM or EEPROM), flash memory, an optical storage device (e.g., CD, DVD, digital optical tape), or other suitable data storage media.

Those skilled in the art will recognize that the embodiments described herein are readily producible using known techniques, materials and equipment. This teaching is presented for purposes of illustration and description but is not intended to be exhaustive or limiting to the forms disclosed. Many modifications and variations will be apparent to those of ordinary skill in the art. The claims below, in contrast, set out its metes and bounds. In the claims, the words "a" and "an" are to be taken to mean "at least one" even if some claim wording explicitly calls for "at least one" or "one or more". In addition any predetermined value, criteria, or rule in the claims may be predetermined at any time up to the time it is required for effective operation unless explicitly stated otherwise.

## Claims

1. A computer-implemented method for producing a protocol comprising:
i. accepting (S107) a time-series of values representative of parameters of a plurality of body locations on a plurality of individuals while those individuals perform a plurality of motion tasks;
ii. analyzing (S112) those values with a data mining computer program running on a computer system to effectively assign at least a portion of the plurality of individuals into one of at least two groups based upon that analyzing of the time-series values, each group being composed of individuals having a common physical attribute;
iii. identifying (S117) one or more subsets of the parameters, and of the associated body locations, and of the associated motion tasks, respectively, that collectively possess effective statistical power to assign an individual to their proper group; and
iv. identifying (S118) rules, that when applied to the subset of the parameters, and of the associated body locations, and of the associated motion tasks properly assigns an individual to their proper group.

2. The method of claim 1 further comprising the step of measuring a time-series of values related to the position of a plurality of body regions on at least one individual of unknown group membership.

3. The method of claim 1 wherein the physical capability is related to a sport skill.

4. The method of claim 2 wherein the physical capability is related to a medical condition.

5. The method of claim 2 wherein the step of measuring comprises tracking a plurality of body positions by three-dimensional image video recording.

6. The method of claim 2 wherein the step of measuring comprises measurements representative of joint angles.

7. The method of claim 1 wherein the at least two groups comprise a group of individuals with back pain and a group of individuals feigning back pain.

8. The method of claim 1 further comprising:
(i) accepting a time-series of values representative of at least a portion of the identified parameters taken from a subject performing at least a portion of the identified motion tasks;
(j) analyzing the accepted values according to at least a portion of the identified rules;
(k) assigning the subject to a group based upon the analyzing of that subject's accepted values.

9. The method of claim 1 wherein_the given physical activity comprises a job-related movement.

10. A computer readable medium having stored thereon the protocol produced by the method of any of claims 1 to 9.

11. An apparatus for producing a protocol, the apparatus configured to:
i. accept (S107) a time-series of values representative of parameters of a plurality of body locations on a plurality of individuals while those individuals perform a plurality of motion tasks;
ii. analyze (S112) those values with a data mining computer program running on a computer system to effectively assign at least a portion of the plurality of individuals into one of at least two groups based upon that analyzing of the time-series values, each group being composed of individuals having a common physical attribute;
iii. identify (S117) one or more subsets of the parameters, and of the associated body locations, and of the associated motion tasks, respectively, that collectively possess effective statistical power to assign an individual to their proper group; and
iv. identify (S118) rules, that when applied to the subset of the parameters, and of the associated body locations, and of the associated motion tasks properly assigns an individual to their proper group.

12. The apparatus of claim 11 further configured to measure a time-series of values related to the position of a plurality of body regions on at least one individual of unknown group membership.

13. The apparatus of claim 11 wherein the physical capability is related to a sport skill.

14. The apparatus of claim 12 wherein the physical capability is related to a medical condition.

15. The apparatus of claim 12 wherein the measuring comprises tracking a plurality of body positions by three-dimensional image video recording.

16. The apparatus of claim 12 wherein the measuring comprises measurements representative of joint angles.

17. The apparatus of claim 11 wherein the at least two groups comprise a group of individuals with back pain and a group of individuals feigning back pain.

18. The apparatus of claim 11 further configured to:
(i) accept a time-series of values representative of at least a portion of the identified parameters taken from a subject performing at least a portion of the identified motion tasks;
(j) analyze the accepted values according to at least a portion of the identified rules;
(k) assign the subject to a group based upon the analyzing of that subject's accepted values.

19. The apparatus of claim 11 wherein_the given physical activity comprises a job-related movement.

## Patentansprüche

1. Computerimplementiertes Verfahren zum Erstellen eines Protokolls, das beinhaltet:
i. Empfangen (S107) einer Zeitreihe von Werten, die für Parameter einer Vielzahl von Körperstellen an einer Vielzahl von Individuen repräsentativ sind, während diese Individuen eine Vielzahl von Bewegungsaufgaben durchführen;
ii. Analysieren (S112) dieser Werte mit einem Datensammlungs-Computerprogramm, das auf einem Computersystem läuft, um wenigstens einen Teil der Vielzahl von Individuen basierend auf dem Analysieren der Zeitreihe-Werte effektiv einer von wenigstens zwei Gruppen zuzuordnen, wobei jede Gruppe aus Individuen besteht, die ein gemeinsames physisches Attribut haben;
iii. Identifizieren (S117) einer oder mehrerer Teilmengen der Parameter bzw. der zugeordneten Körperstellen bzw. der zugeordneten Bewegungsaufgaben, die kollektiv effektive statistische Aussagekraft haben, um ein Individuum seiner geeigneten Gruppe zuzuordnen; und
iv. Identifizieren (S118) von Regeln, die, wenn sie auf die Teilmenge der Parameter und der zugeordneten Körperstellen und der zugeordneten Bewegungsaufgaben angewendet werden, ein Individuum ordnungsgemäß seiner geeigneten Gruppe zuordnen.

2. Verfahren nach Anspruch 1, das des Weiteren den Schritt des Messens einer Zeitreihe von Werten beinhaltet, die sich auf die Position einer Vielzahl von Körperbereichen an wenigstens einem Individuum unbekannter Gruppenzugehörigkeit beziehen.

3. Verfahren nach Anspruch 1, wobei sich die physische Leistungsfähigkeit auf eine sportliche Fähigkeit bezieht.

4. Verfahren nach Anspruch 2, wobei sich die physische Leistungsfähigkeit auf einen medizinischen Zustand bezieht.

5. Verfahren nach Anspruch 2, wobei der Schritt des Messens das Verfolgen einer Vielzahl von Körperpositionen durch dreidimensionale Videobildaufzeichnung beinhaltet.

6. Verfahren nach Anspruch 2, wobei der Schritt des Messens Messungen beinhaltet, die für Gelenkwinkel repräsentativ sind.

7. Verfahren nach Anspruch 1, wobei die wenigstens zwei Gruppen eine Gruppe von Individuen mit Rückenschmerzen und eine Gruppe von Individuen, die Rückenschmerzen vorgeben, aufweisen.

8. Verfahren nach Anspruch 1, das des Weiteren beinhaltet:
(i) Empfangen einer Zeitreihe von Werten, die für wenigstens einen Teil der identifizierten Parameter repräsentativ sind, die von einer Testperson genommen wurden, welche wenigstens einen Teil der identifizierten Bewegungsaufgaben durchführt;
(j) Analysieren der empfangenen Werte gemäß wenigstens einem Teil der identifizierten Regeln;
(k) Zuordnen der Testperson zu einer Gruppe basierend auf dem Analysieren der für diese Testperson empfangenen Werte.

9. Verfahren nach Anspruch 1, wobei die gegebene physische Aktivität eine berufsbezogene Bewegung aufweist.

10. Computerlesbares Medium, auf dem das Protokoll gespeichert ist, das mit dem Verfahren nach einem der Ansprüche 1 bis 9 erstellt worden ist.

11. Vorrichtung zum Erstellen eines Protokolls, wobei die Vorrichtung konfiguriert ist zum:
i. Empfangen (S107) einer Zeitreihe von Werten, die für Parameter einer Vielzahl von Körperstellen an einer Vielzahl von Individuen repräsentativ sind, während diese Individuen eine Vielzahl von Bewegungsaufgaben durchführen;
ii. Analysieren (S112) dieser Werte mit einem Datensammlungs-Computerprogramm, das auf einem Computersystem läuft, um wenigstens einen Teil der Vielzahl von Individuen basierend auf dem Analysieren der Zeitreihe-Werte effektiv einer von wenigstens zwei Gruppen zuzuordnen, wobei jede Gruppe aus Individuen besteht, die ein gemeinsames physisches Attribut haben;
iii. Identifizieren (S117) einer oder mehrerer Teilmengen der Parameter bzw. der zugeordneten Körperstellen bzw. der zugeordneten Bewegungsaufgaben, die kollektiv effektive statistische Aussagekraft haben, um ein Individuum seiner geeigneten Gruppe zuzuordnen; und
iv. Identifizieren (S118) von Regeln, die, wenn sie auf die Teilmenge der Parameter und der zugeordneten Körperstellen und der zugeordneten Bewegungsaufgaben angewendet werden, ein Individuum ordnungsgemäß seiner geeigneten Gruppe zuordnen.

12. Vorrichtung nach Anspruch 11, die des Weiteren dazu konfiguriert ist, eine Zeitreihe von Werten zu messen, die sich auf die Position einer Vielzahl von Körperbereichen an wenigstens einem Individuum unbekannter Gruppenzugehörigkeit beziehen.

13. Vorrichtung nach Anspruch 11, wobei sich die physische Leistungsfähigkeit auf eine sportliche Fähigkeit bezieht.

14. Vorrichtung nach Anspruch 12, wobei sich die physische Leistungsfähigkeit auf einen medizinischen Zustand bezieht.

15. Vorrichtung nach Anspruch 12, wobei das Messen das Verfolgen einer Vielzahl von Körperpositionen durch dreidimensionale Bildvideoaufzeichnung beinhaltet.

16. Vorrichtung nach Anspruch 12, wobei das Messen Messungen beinhaltet, die für Gelenkwinkel repräsentativ sind.

17. Vorrichtung nach Anspruch 11, wobei die wenigstens zwei Gruppen eine Gruppe von Individuen mit Rückenschmerzen und eine Gruppe von Individuen, die Rückenschmerzen vorgeben, aufweisen.

18. Vorrichtung nach Anspruch 11, die des Weiteren konfiguriert ist zum:
(i) Empfangen einer Zeitreihe von Werten, die für wenigstens einen Teil der identifizierten Parameter repräsentativ sind, die von einer Testperson genommen wurden, welche wenigstens einen Teil der identifizierten Bewegungsaufgaben durchführt;
(j) Analysieren der empfangenen Werte gemäß wenigstens einem Teil der identifizierten Regeln;
(k) Zuordnen der Testperson zu einer Gruppe basierend auf dem Analysieren der für diese Testperson empfangenen Werte.

19. Vorrichtung nach Anspruch 11, wobei die gegebene physische Aktivität eine berufsbezogene Bewegung aufweist.

## Revendications

1. Procédé mis en oeuvre par ordinateur pour produire un protocole, consistant à :
i. accepter (S107) une série temporelle de valeurs représentatives de paramètres d'une pluralité de positions corporelles sur une pluralité d'individus tandis que ces individus effectuent une pluralité de tâches de mouvement ;
ii. analyser (S112) ces valeurs avec un programme d'ordinateur d'exploration de données fonctionnant sur un système d'ordinateur pour attribuer efficacement au moins une partie de la pluralité d'individus dans l'un d'au moins deux groupes à partir de cette analyse des valeurs de la série temporelle, chaque groupe étant composé d'individus ayant un attribut physique commun ;
iii. identifier (S117) un ou plusieurs sous-ensembles des paramètres, les positions corporelles associées, et les tâches de mouvement associées, respectivement, qui possèdent collectivement une puissance statistique effective pour attribuer un individu à leur propre groupe ;
et
iv. identifier (S118) des règles qui, lorsqu'elles sont appliquées au sous-ensemble des paramètres, et des positions corporelles associées, et des tâches de mouvement associées attribuent correctement un individu à leur propre groupe.

2. Procédé selon la revendication 1, comprenant en outre l'étape consistant à mesurer une série temporelle de valeurs liées à la position d'une pluralité de régions corporelles sur au moins un individu membre d'un groupe inconnu.

3. Procédé selon la revendication 1, dans lequel l'aptitude physique est liée à une habileté sportive.

4. Procédé selon la revendication 2, dans lequel l'aptitude physique est liée à un état de santé.

5. Procédé selon la revendication 2, dans lequel l'étape de mesure consiste à pister une pluralité de positions corporelles par un enregistrement vidéo tridimensionnel.

6. Procédé selon la revendication 2, dans lequel l'étape de mesure comprend des mesures représentatives d'angles joints.

7. Procédé selon la revendication 1, dans lequel les au moins deux groupes comprennent un groupe d'individus avec une douleur dorsale et un groupe d'individus feignant une douleur dorsale.

8. Procédé selon la revendication 1, consistant en outre à :
(i) accepter une série temporelle de valeurs représentatives d'au moins une partie des paramètres identifiés pris à partir d'un sujet effectuant au moins une partie des tâches de mouvement identifiées ;
(j) analyser les valeurs acceptées en fonction d'au moins une partie des règles identifiées ;
(k) attribuer le sujet à un groupe à partir de l'analyse des valeurs acceptées pour ce sujet.

9. Procédé selon la revendication 1, dans lequel l'activité physique donnée comprend un mouvement associé à un métier.

10. Médium lisible par un ordinateur, contenant le protocole produit le procédé selon l'une quelconque des revendications 1 à 9.

11. Appareil destiné à produire un protocole, l'appareil étant conçu pour :
i. accepter (S107) une série temporelle de valeurs représentatives de paramètres d'1 une pluralité de positions corporelles sur une pluralité d'individus tandis que ces individus effectuent une pluralité de tâches de mouvement ;
ii. analyser (S112) ces valeurs avec un programme d'ordinateur d'exploration de données fonctionnant sur un système d'ordinateur pour attribuer efficacement au moins une partie de la pluralité d'individus dans l'un d'au moins deux groupes à partir de cette analyse des valeurs de la série temporelle, chaque groupe étant composé d'individus ayant un attribut physique commun ;
iii. identifier (S117) un ou plusieurs sous-ensembles des paramètres, les positions corporelles associées, et les tâches de mouvement associées, respectivement, qui possèdent collectivement une puissance statistique effective pour attribuer un individu à leur propre groupe ;
et
iv. identifier (S118) des règles qui, lorsqu'elles sont appliquées au sous-ensemble des paramètres, et des positions corporelles associées, et des tâches de mouvement associées attribuent correctement un individu à leur propre groupe.

12. Appareil selon la revendication 11, conçu en outre pour mesurer une série temporelle de valeurs liées à la position d'une pluralité de régions corporelles sur au moins un individu membre d'un groupe inconnu.

13. Appareil selon la revendication 11, dans lequel l'aptitude physique est liée à une habileté sportive.

14. Appareil selon la revendication 12, dans lequel l'aptitude physique est liée à un état de santé.

15. Appareil selon la revendication 12, dans lequel l'étape de mesure consiste à pister une pluralité de positions corporelles par un enregistrement vidéo tridimensionnel.

16. Appareil selon la revendication 12, dans lequel l'étape de mesure comprend des mesures représentatives d'angles joints.

17. Appareil selon la revendication 11, dans lequel les au moins deux groupes comprennent un groupe d'individus avec une douleur dorsale et un groupe d'individus feignant une douleur dorsale.

18. Appareil selon la revendication 11, consistant en outre à :
(i) accepter une série temporelle de valeurs représentatives d'au moins une partie des paramètres identifiés pris à partir d'un sujet effectuant au moins une partie des tâches de mouvement identifiées ;
(j) analyser les valeurs acceptées en fonction d'au moins une partie des règles identifiées ;
(k) attribuer le sujet à un groupe à partir de l'analyse des valeurs acceptées pour ce sujet.

19. Appareil selon la revendication 11, dans lequel l'activité physique donnée comprend un mouvement associé à un métier.
